# FASCICULE DE BREVET EUROPEEN

(11) **EP 2 131 831 B1**
(45) Date de publication et mention de la délivrance du brevet: **15.05.2013**
(21) Numéro de dépôt: 08775664.9
(22) Date de dépôt: 03.03.2008
(51) Int. Cl.: A61K 31/785, A61K 31/16, A61K 47/48, A61P 31/04, A61P 31/06, A61P 31/10

(54) **CONJUGUES D'ACIDES GRAS ET POLY-L-LYSINE POUR LUTTER CONTRE DES MICROORGANISMES PATHOGENES**
KONJUGIERTE FETTSÄUREN UND POLY-L-LYSIN ZUR BEKÄMPFUNG KRANKHEITSERREGENDER MIKROORGANISMEN
CONJUGATED FATTY ACIDS AND POLY-L-LYSINE FOR COMBATING PATHOGENIC MICRO-ORGANISMS

(30) Priorité: 06.03.2007 FR 0753675
(43) Date de publication de la demande: 16.12.2009
(73) Titulaire: Geffard, Michel, 33400 Talence (FR)
(72) Inventeur: Geffard, Michel, 33400 Talence (FR)
(74) Mandataire: Cenatiempo, Julie Adeline Anne
(86) Numéro de dépôt international: PCT/FR2008/050361
(87) Numéro de publication internationale: WO 2008/122741

(56) Documents cités:
- WO-A-96/15810
- WO-A-2006/035431
- SHIMA S ET AL: "ANTIMICROBIAL ACTION OF EPSILON-POLY-L-LYSINE" JOURNAL OF ANTIBIOTICS, JAPAN ANTIBIOTICS RESEARCH ASSOCIATION, TOKYO, JP, vol. 37, no. 11, 1984, pages 1449-1455, XP009079625 ISSN: 0021-8820
- DELIHAS ET AL.: "high sensitivity of Mycobacterium species to the bactericidas activity by polylysine" FEMS MICROBIOLOGY LETTERS, vol. 132, 1995, pages 233-237, XP002453102

## Description

La présente invention se rapporte à l'utilisation de poly-L-Lysine greffée avec au moins un acide gras ou dérivé d'acide gras, pour lutter contre des bactéries, des levures et des champignons pathogènes.

Les microorganismes pathogènes sont à l'origine de multiples maladies infectieuses, responsables de nombreux décès dans le monde.

La découverte des antibiotiques a permis de lutter efficacement contre certaines de ces maladies bactériennes et mycosiques.

Les antibiotiques sont des substances naturelles produites par des bactéries du sol, certains champignons ou des produits de synthèse, qui à faibles concentrations, agissent sur d'autres microorganismes en les tuant ou en ralentissant leur croissance, sans être toxiques pour l'homme ou l'animal. Toutefois l'usage massif des antibiotiques a engendré des phénomènes de résistance aux bactéries, levures et champignons pathogènes, les microorganismes s'adaptant aux changements environnementaux par leur multiplication rapide.

En outre, plus la résistance est élevée, plus on prescrit des antibiotiques nouveaux, ce qui a favorisé l'émergence de nouvelles résistances aux anti-bactériens et anti-fongiques classiques et entraîné l'apparition des maladies nosocomiales ou la résurgence de maladies infectieuses telle que la tuberculose. La demande W02006/035431 décrit des agents antimicrobiens, notamment des agents polymériques constitués de lysine et d'acide gras, mais ceux-ci ne sont pas suffisamment efficaces comme médicaments anti-microorganismes pathogènes.

Il est donc impératif de trouver et développer de nouveaux systèmes capables de lutter efficacement contre les microorganismes pathogènes, en vue d'éradiquer les maladies infectieuses dont ils sont responsables.

Cest l'objectif de la présente invention qui se propose, pour y répondre, d'utiliser des acides gras greffés sur un polypeptide spécifique.

En particulier, la présente invention vise l'utilisation d'au moins un conjugué entre au moins un acide gras ou dérivé d'acide gras, et au moins une poly-L-Lysine, pour fabriquer un médicament destiné à lutter contre des microorganismes pathogènes.

Des conjugués de polylysine sont déjà connus pour d'autres applications thérapeutiques, comme décrit dans WO96/15810 mais aucunement le conjugué objet de la présente invention pour lutter contre des microorganismes pathogènes.

L'invention est maintenant décrite en détail.

Par microorganismes pathogènes on entend des bactéries, des levures et/ou des champignons, responsables d'infections humaines ou animales.

Par poly-L-Lysine, on entend une poly-L-Lysine linéaire ou ramifiée. Avantageusement les conjugués selon l'invention sont capables de détruire ou de ralentir la croissance des microorganismes pathogènes.

On suppose que la poly-L-Lysine s'absorbe sur certaines parois cellulaires et est ensuite internalisée avec les composés acides gras spécifiques interférant dans le métabolisme des microorganismes. L'effet bactéricide propre de la poly-L-Lysine linéaire ou ramifiée est amplifié par la présence des acides gras.

Selon un autre avantage les acides gras du conjugué utile selon l'invention ne sont pas métabolisés lorsqu'ils sont administrés à l'homme ou à l'animal. Les conjugués circulent dans l'organisme jusqu'à fixation sur le microorganisme ciblé.

En fonction de l'activité spécifique recherchée, les conjugués utiles selon l'invention sont réalisés avec des acides gras adaptés à un effet particulier. C'est la conformation spécifique des acides gras utilisés avec la poly-L-Lysine qui donne l'effet bactéricide, anti-mycosique ou anti-lévurien au conjugué.

Des conjugués préférés pour la présente invention sont des conjugués monofonctionnels c'est-à-dire des conjugués de la poly-L-Lysine avec un seul acide gras ou dérivé d'acide gras, et des conjugués bifonctionnels c'est-à-dire des conjugués de la poly-L-Lysine avec deux acides gras et/ou dérivés d'acide gras différents.

Parmi les conjugués monofonctionnels particulièrement adaptés pour la présente invention on peut citer notamment l'Acide Acétique-poly-L-Lysine ou acétyl-poly-L-Lysine, l'Acide Pyruvique-poly-L-Lysine ou pyruvyl-poly-L-Lysine, l'Acide Propionique-poly-L-Lysine ou propionyl-poly-L-Lysine, l'Acide Butyrique-poly-L-Lysine ou butyryl-poly-L-Lysine, l'Acide Lactique-poly-L-Lysine ou lactyl-Poly-L-Lysine, l'Acide Succinique-poly-L-Lysine ou succinyl-poly-L-Lysine, l'Acide glutarique-poly-L-Lysine ou glutaryl-poly-L-Lysine, l'Acide caprdique-poly-L-Lysine ou caproyl-poly-L-Lysine, l'Acide caprylique-poly-L-Lysine ou caprylyl-poly-L-Lysine l'Acide caprique-poly-L-Lysine ou capryl-poly-L-Lysine, l'Acide Azélaique poly-L-Lysine ou azélayl-poly-L-Lysine, l'Acide Laurique-poly-L-Lysine ou lauryl-poly-L-Lysine, l'Acide Myrisitique-poly-L-Lysine ou myristyl-poly-L-Lysine, l'Acide Palmitique-poly-L-Lysine ou palmityl-poly-L-Lysine, l'Acide Palmitoléique-poly-L-Lysine ou palmitoleyl-poly-L-Lysine, ou encore l'Acide Oléique-poly-L-Lysine ou oléyl-poly-L-Lysine.

A titre d'exemple, le conjugué lauryl-poly-L-Lysine est particulièrement actif sur les staphylocoques, et le conjugué pyruvyl-poly-L-Lysine est particulièrement actif sur les streptocoques, les citrobacters et/ou les levures telle que Candida albicans par exemple.

Parmi les conjugués bifonctionnels particulièrement adaptés pour la présente invention on peut citer à titre d'exemple le capryl-poly-L-Lysine-laurique ou le farnésyl-poly-L-Lysine Oléique.

Selon un aspect de l'invention, on utilise préférentiellement des concentrations en acide gras ou dérivés d'acides gras pour réaliser les conjugués de l'ordre de 20 à 100 milligrammes/litre. Ces faibles concentrations ont pour avantage de conduire à des produits stables et non toxiques.

Les conjugués selon l'invention peuvent être utilisés pour fabriquer des médicaments pour lutter contre des infections en général.

Un médicament particulièrement efficace présentant une large activité bactéricide comprendrait par exemple une association de pyruvyl-poly-L-Lysine à 5X10⁻³Molaire et de lauryl-poly-L-Lysine-Capryl à 5×10⁻⁴Molaire.

Les conjugués selon l'invention peuvent également être utilisés en cosmétologie. Les conjugués utiles selon l'invention peuvent être synthétisés selon le schéma réactionnel suivant, illustré avec l'acide laurique :

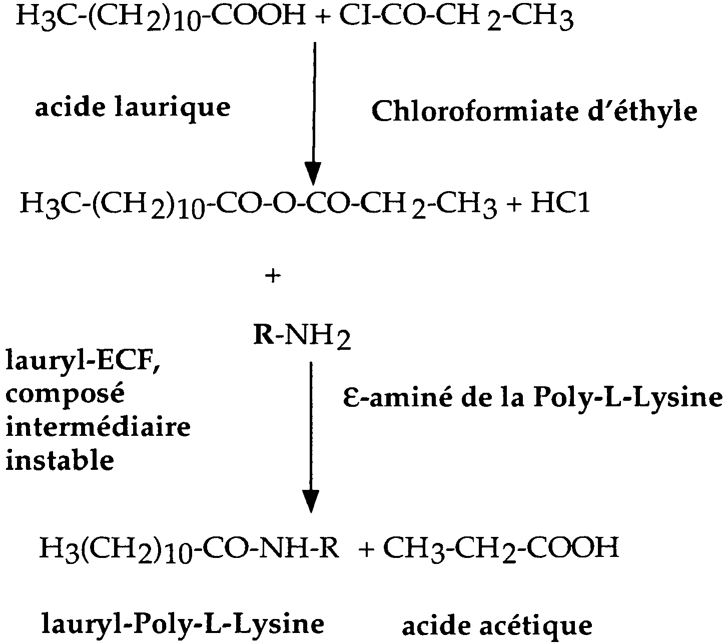

Les acides gras ou dérivés d'acides gras n'étant pas solubles en milieux aqueux, ils sont repris dans un solvant organique, par exemple et préférentiellement le méthanol anhydre.

On adjoint aux acides gras du chloroformiate d'éthyle qui active leur groupement carboxylique.

Le composé d'activation obtenu est ensuite mis en présence sous agitation d'une solution aqueuse contenant de la poly-L-Lysine, ce qui provoque immédiatement une réaction d'amidification.

On obtient ainsi un conjugué acide(s) gras-poly-L-Lysine, ou dérivé(s) d'acide(s) gras-poly-L-Lysine qui est finalement purifié par dialyse pour éliminer les solvants, l'excès d'acide(s) gras et les produits secondaires de la réaction d'amidification.

Ce conjugué acide(s) gras-poly-L-Lysine, ou dérivé(s) d'acide(s) gras-poly-L-Lysine peut éventuellement être associé à un ou plusieurs conjugués antioxydant(s)-poly-L-Lysine et/ou à un ou plusieurs conjugués acide(s) aminé(s)-poly-L-Lysine. La solution obtenue a également un effet bactéricide.

Des essais ont été effectués pour montrer l'efficacité des conjugués utiles selon l'invention.

Différentes souches de microorganismes ont été utilisées pour ces essais :
- des souches bactériennes Gram⁻ :
   - Citrobacter koserii
   - Citrobacter diversus
   - Escherichia coli (plusieurs sérotypes)
   - Klebsiella pneumoniae
   - Klebsiella oxytoca
   - Morganella morganii
   - Proteus mitabilis
   - Pseudomonas aeruginosa
   - Pseudomonas putida
   - Serratia marcescens
   - Alcalescens diaspar
   - Enterobacter hafniae
- des souches bactériennes Gram⁺ :
   - Staphylococcus aureus
   - Streptococcus epidermis
   - Streptococcus A, B, D
   - Mycobacterium tuberculosis
- des souches bactériennes multirésistantes aux antibiotiques (Staphylococcus aureus, Alcalescens dispar , Escherichia coli, Staphylococcus epidermis, Pseudomonas aeruginosa),
- des levures Candida albicans de 6 sous-groupes différents.

Les différents essais ont été réalisés selon le mode opératoire décrit en suivant. Les effets anti-bactéricides et anti-fongiques des solutions testées ont été évalués sur des boîtes contenant des milieux de cultures appropriés à la croissance :
- gélose chocolat haemophilus pour la culture de Neisseria,
- gélose BCP pour les germes peu exigeants,
- gélose Mueller Hinton 2 (MH2-F) pour la majorité des souches bactériennes sauf Neisseria et les streptocoques,
- gélose columbia ANC (COL ANC-F) pour les streptocoques,
- gélose Sabouraud gentamicine chloramphénicol (SAB GENTA CHL-F) pour les candidas albicans et les levures en général, et
- milieu Leowenstein, Coletsos pour Mycobacterium tuberculosis.

Les boîtes sont recouvertes par 1ml de solution bactérienne ou mycosique à la concentration de 0,5 à 1 de Mac Farland (lecture au densitomètre) soit 1,5×10⁸ bactéries/ml.

La boîte est ensuite séchée 15 minutes à 37°, et on applique 2 gouttes (50µl) de chaque solution à tester.

La boîte de culture est recouverte après 16 heures d'incubation à 37°.

Chaque plage de lyse due à l'activité anti-bactérienne ou anti-fongique des conjugués est mesurée. La lecture de l'effet bactéricide ou antifongique est mesurée en cm².

Les résultats de différents tests sont présentés ci-dessous.

### - 1 - Comparaison de l'activité bactéricide des coniugués selon l'invention avec celle d'acides gras libres

Les résultats sont évalués visuellement selon l'échelle suivante :
0 pas d'activité, culture négative
+ correspond à 10³ colonies
++ correspond à 10⁴ colonies
+++ correspond à 10⁶ colonies, et
++++ correspond à 10⁷ colonies.

Ils sont présentés dans le tableau ci-dessous :

| Longueur de la chaîne | Nom de l'acide gras non lié | Activité bactéricide sur Escherichia coli ou Staphylococcus aureus de l'acide gras seul | Activité bactéricide sur Escherichia coli ou Staphylococcus aureus du conjugué |
|---|---|---|---|
| C2-C4 | C2 = acetate | 0 | +++ |
| | C3 = propionate | 0 | +++ |
| | C4 = butyrate | 0 | +++ |
| | C5 = acide valérique | 0 | +++ |
| C6 | Acide Caproïque | ++ | +++ |
| C8 | Acide Caprylique | +++ | +++ |
| C10 | Acide Caprique | ++++ | +++ |
| C12 | Acide Laurique | ++++ | ++ |
| C14 | Acide Myristique | + | + |
| C16 | Acide Palmitique | 0 | + |
| C16 : 1 | Acide Palmitoléique | ++ | +++ |
| C18 : 1 | Acide Oléique | + | + |
| Farcys | Farnesyl-cystéine | 0 | ++ |

### - 2 - Efficacité de conjugués d'acides gras et poy-L-Lysine sur certains microorganismes

La concentration en acides gras sur la poly-L-Lysine est de 5×10⁻³M.

| | Lactyl-poly-L-Lysine (H₃c-CHOH-CO-NH-PL) | Propionylpoy-L-lysine (CH₃-CH₂-CO-PL) | Pyruvyl-Poly-L-Lysine (H₃C-CO-CO-PL) | Butyryl- poly-L-Lysine (H₃C-CH₂-CH₂-CO-PL) | Acétyl- poly-L-Lysine (H₃C-CO-PL) |
|---|---|---|---|---|---|
| Staphylococcus aureus. | 1,82 cm² | 2,3 cm² | 2,13 cm² | 1,82 cm² | 1,68 cm² |
| Escherichia coli. | 1,92 cm² | | 2,09 cm² | 2,65 cm² | 0 |
| Pseudomonas aeruginosa. | 2,34 cm² | 0 | 1,32 cm² | | |
| Candida albicans. | 1,05cm² | 2,13cm² | 2,46 cm² | 2,3 cm² | 1,97cm² |
| Citro bacter koserii. | 1,13 cm² | 0 | 1,54 cm² | 1,77 cm² | 1,77 cm^{z} |
| Klebsiella oxytoca. | 0 | 0 | 1,33 cm² | 0 | 3,14 cm² |
| Streptocoque B. | peu sensible | 0 | 1,77cm² qqls colonies | 1,33 cm² qqls colonies | 3,14 cm² colonies |
| Streptocoque D. | 2,01 cm² | 0 | 2,27 cm² | 0 | 4,15cm² |
| Enterobacter Hafniae | 1,44cm² | 1,12 cm² | 2,27 cm² | 2,03 cm² | 2,71 cm² |
| Alcalescens Diaspar. | 1,53cm² | 1,13cm^{2 inhibition partielle} | 1,53cm² | 0,85 cm² | 1,4cm² |
| Morganella Morganii. | 0 | 0 | 0 | 0 | 0 |
| Proteus Mirabilis. | 0 | 0 | 0 | 0 | 0 |
| Nesseria | 1,65cm² | 1,13 cm² | 2,01cm² | 0,78 cm² | 2,27cm² |

### - 3 - Comparaison de l'activité bactéricide des conjugués selon l'invention avec celle d'acides gras coniugués avec une poly-Ornithine (ORN)

Les solutions testées sont à la même concentration en acides gras que celles avec les conjugués poly-L-Lysine, soit 5×10⁻³ d'acides gras liés.

| | Lactyl-ORN | Pyruvyl-ORN | Butyryl-ORN | Acétyl-ORN |
|---|---|---|---|---|
| Staphylococcu s aureus. | 0 | 0 | 0 | 0 |
| Pseudomonas aeruginosa. | 0 | 0 | 0 | 0 |
| Escherichia coli. | 0 | 0 | 0 | 0 |

Ces résultats montrent bien que c'est la conformation des acides gras liés aux acides aminés de la poly-L-Lysine qui donne l'effet bactéricide et anti-mycosique.

### - 4 - Efficacité d'un mélange de conjugués selon l'invention sur des souches multi-résistantes

Les exemples de bactéricidie sont obtenus avec la préparation capryl-poly-L-Lysine-lauryl et pyruvyl-poly-L-Lysine à différentes concentrations : à 5×10⁻⁴M (5D4), à 5×10⁻³M (5D3) et à 5×10⁻²M (5D2).

| | Pyruvyl-Poly-L-Lysine (5D4) + Capryl-Poly-L-Lysine-Lauryl (5D4) | Pyruvyl-Poly-L-Lysine (5D3) + Capryl-Poly-L-Lysine-Lauryl (5D4) | Pyruvyl-Poly-L-Lysine (5D3) + Capryl-Poly-L-Lysine-Lauryl (5D2) |
|---|---|---|---|
| Staphylococcus aureus. | 1,33 cm² | 1,65 cm² | 2,27 cm² |
| Escherichia coli. | Non testé | 1,54 cm² | 1,77 cm² |
| Pseudomonas spp | Non testé | 2,86 cm² | Non testé |
| Staphylococcus epidermidis | Non testé | 2,54cm² | Non testé |

Plus de 15 souches multirésistantes prélevées sur des malades ont toutes été sensibles à ces préparations de mélanges de conjugués selon l'invention.

### - 5 - Efficacité de l'activité bactéricide des conjugués selon l'invention sur la souche Mycobacterium tuberculosis

La souche Mycobacterium tuberculosis est cultivée pendant un mois et demi sur milieu de Loewenstein Coletsos : des colonnies apparaissent à la concentration de 10⁶.

On met en contact du Mycobacterium tuberculosis une solution pyruvyl-poly-L-Lysine à 5×10⁻³M + capryl-poly-L-Lysine-Lauryl à 5×10⁻⁴M.

On obtient une culture négative.

La solution contenant le mélange de conjugués selon l'invention a donc un effet bactéricide sur Mycobacterium tuberculosis.

### - 6 - Activité bactéricide des conjugués selon l'invention

On ajoute à 1mL de pyruvyl-poly-L-Lysine à 5×10⁻³M + capryl-poly-L-Lysine-Lauryl à 5×10⁻⁴M, les souches suivantes :
- Staphylococcus aureus (densité de Mac Farland = 4, 1,2×10⁹ bactéries/ml)
- Escherichia coli (densité de Mac Farland = 2, 6×10⁸ bactéries/ml)
- Pseudomonas aeruginosa (densité de Mac Farland = 2, 6×10⁸ bactéries/ml).

Les mesures de l'activité bactéricide après 1 heure, 3 heures, 5 heures et 24 heures de contact sont répertoriées dans le tableau suivant :

| | Staphylococcus aureus | Escherichia coli | Pseudomonas aeruginosa |
|---|---|---|---|
| 1 heure | +++ 10⁶ | +++ 10⁶ | +++ 10⁶ |
| 3 heures | Négative | + 10³ | +++ 10⁶ |
| 5 heures | Négative | Négative | + 10³ |
| 24 heures | Négative | Négative | Négative |

### - 7 - Evaluation de la concentration minimale inhibitrice des conjugués selon l'invention

Sur une souche multirésistante de staphylococcus epidermidis, la concentration minimale inhibitrice pour une solution capryl-poly-L-Lysine-lauryl + pyruvate-poly-L-Lysine est :
- inférieure à 1,25×10⁵M pour capryl-poly-Lysine-lauryl, et
- inférieure à 1,25×10⁴M pour pyruvate-poly-Lysine.

## Revendications

1. Utilisation d'au moins un conjugué entre au moins un acide gras ou dérivé d'acide gras, et au moins une poly-L-Lysine, pour fabriquer un médicament destiné à lutter contre des microorganismes pathogènes.

2. Utilisation selon la revendication 1, **caractérisée en ce que** le conjugué est obtenu par la mise en oeuvre d'un procédé comprenant notamment les étapes suivantes :
- adjonction de chloroformiate à au moins un acide gras ou dérivé d'acides gras,
- mise en présence du composé obtenu d'une solution aqueuse contenant de la poly-L-Lysine,
- réaction d'amidification et obtention du conjugué acide(s) gras - poly-L-Lysine ou dérivé(s) d'acide(s) gras-poly-L-Lysine.

3. Utilisation selon l'une des revendications précédentes, **caractérisée en ce que** le ou les acide(s) gras ou dérivé(s) d'acide(s) gras sont utilisés dans des concentrations comprises entre 20 à 100 milligrammes/litre pour réaliser les conjugués.

4. Utilisation selon l'une quelconque des revendications précédentes, **caractérisée en ce que** au moins un conjugué est le conjugué lauryl-poly-L-Lysine pour fabriquer un médicament destiné à lutter contre des staphylocoques.

5. Utilisation selon l'une quelconque des revendications 1 à 4, **caractérisée en ce que** au moins un conjugué est le conjugué pyruvyl-poly-Lysine pour fabriquer un médicament destiné à lutter contre des citrobacters et/ou des streptocoques et/ou des levures.

6. Utilisation selon l'une quelconque des revendications précédentes, **caractérisée en ce que** l'on associe un conjugué lauryl-poly-L-Lysine-Capryl et un conjugué pyruvyl-poly-L-Lysine pour fabriquer un médicament destiné à lutter contre Mycobacterium tuberculosis.

7. Utilisation selon l'une quelconque des revendications 1 à 6, **caractérisée en ce que** le au moins un conjugué est associé à un ou plusieurs conjugués anti-oxydant(s)-poly-L-Lysine et/ou à un ou plusieurs conjugués acide(s) aminé(s)-poly-L-Lysine.

## Patentansprüche

1. Verwendung von wenigstens einer Konjugation zwischen wenigstens einer Fettsäure oder wenigstens einem Fettsäurederivat und wenigstens einem Poly-L-Lysin zur Herstellung eines Arzneimittels, um pathogene Mikroorganismen zu bekämpfen.

2. Verwendung nach Anspruch 1, **dadurch gekennzeichnet, dass** die Konjugation durch die Ausführung eines Verfahrens erhalten wird, das insbesondere die folgenden Schritte aufweist:
- Hinzufügen von Chlorameisensäureester zu wenigstens einer Fettsäure oder einem Fettsaurederivat;
- Zusammenbringen der erhaltenen Zusammensetzung und einer Poly-L-Lysin enthaltenden wässrigen Lösung;
- Amidifizierungsreaktion und Erhalten der Konjugation Fettsäure(n)-Poly-L-Lysin oder Fettsäurederivat(e)-Poly-L-Lysin.

3. Verwendung nach einem der vorhergehenden Ansprüche, **dadurch gekennzeichnet, dass** die Fettsäure(n) oder das(die) Fettsäurederivat(e) in Konzentrationen zwischen 20 und 100 Milligramm/Liter zum Herstellen der Konjugation verwendet werden.

4. Verwendung nach einem der vorhergehenden Ansprüche, **dadurch gekennzeichnet, dass** wenigstens eine Konjugation die Konjugation Lauryl-Poly-L-Lysin zur Herstellung eines Medikaments ist, um Staphylokokken zu bekämpfen.

5. Verwendung nach einem der Ansprüche 1 bis 4, **dadurch gekennzeichnet, dass** wenigstens eine Konjugation die Konjugation Pyruvyl-Poly-Lysin zur Herstellung eines Arzneimittels ist, um Citrobacter und/oder Streptokokken und/oder Hefen zu bekämpfen.

6. Verwendung nach einem der vorhergehenden Ansprüche, **dadurch gekennzeichnet, dass** eine Konjugation Lauryl-Poly-L-Lysin-Capryl und eine Konjugation Pyruvyl-Poly-L-Lysin zur Herstellung eines Arzneimittels verbunden werden, um Mycobacterium tuberculosis zu bekämpfen.

7. Verwendung nach einem der Ansprüche 1 bis 6, **dadurch gekennzeichnet, dass** die wenigstens eine Konjugation mit einer oder mehreren Konjugation Antioxidans(-tien)-Poly-L-Lysin und/oder mit einer oder mehreren Konjugationen Aminosäure(n)-Poly-L-Lysin verbunden ist.

## Claims

1. Use of at least one conjugate between at least one fatty acid or fatty acid derivative, and at least one poly-L-lysine, in order to manufacture a drug intended to combat pathogenic microorganisms.

2. Use according to claim 1, **characterised in that** the conjugate is obtained by implementing a method comprising in particular the following steps:
- addition of chloroformate to at least one fatty acid or fatty acid derivative,
- adding an aqueous solution containing poly-L-lysine to the compound obtained,
- an amidification reaction and obtaining of the fatty acids(s) - poly-L-lysine or fatty acid derivative(s)
- poly-L-lysine conjugate.

3. Use according to one of the preceding claims, **characterised in that** the fatty acid(s) or fatty acid derivative(s) are used in concentrations of between 20 and 100 milligrams/litre in order to produce the conjugates.

4. Use according to any one of the preceding claims, **characterised in that** at least one conjugate is the lauryl-poly-L-lysine conjugate for manufacturing a drug intended to combat staphylococci.

5. Use according to any one of claims 1 to 4, **characterised in that** at least one conjugate is the pyruvyl-poly-lysine conjugate for manufacturing a drug intended to combat citrobacters and/or streptococci and/or yeasts.

6. Use according to any one of the preceding claims, **characterised in that** a lauryl-poly-L-lysine-capryl conjugate and a pyruvyl-poly-L-lysine conjugate are associated for manufacturing a drug intended to combat Mycobacterium tuberculosis.

7. Use according to any one of claims 1 to 6, **characterised in that** the at least one conjugate is associated with one or more antioxidant-poly-L-lysine conjugates and/or one or more amino acid-poly-L-lysine conjugates.
